# EUROPEAN PATENT APPLICATION

(11) **EP 4 465 049 A1**
(43) Date of publication of application: **20.11.2024**
(21) Application number: 23174037.4
(22) Date of filing: 17.05.2023
(51) Int. Cl.: G01N 33/68, G01N 33/50, B01L 1/00, G01N 1/36, G01N 15/02

(54) **METHOD AND DEVICE FOR ASSAYING A PLURALITY OF BIOLOGICAL SAMPLES**

(71) Applicant: Leica Microsystems CMS GmbH, 35578 Wetzlar (DE)
(72) Inventor: Alsheimer, Soeren, 35578 Wetzlar (DE); Bradl, Joachim, 35578 Wetzlar (DE)
(74) Representative: Schaumburg und Partner Patentanwälte mbB

(57) **Abstract**

A method for assaying a plurality of biological samples (108) comprises the following steps: Preparing a plurality of discrete entities (100, 1800), each discrete entity (100, 1800) comprising a polymeric compound, at least one of the biological samples (108) and a marker (110). Arranging the plurality of discrete entities (100, 1800) in an imaging container (102). Generating at least one first optical read-out of at least one discrete entity (100, 1800) of the plurality of discrete entities (100, 1800). Determining a first representation of the marker (110) and at least one characteristic related to the biological sample (108) for at least one discrete entity (100, 1800) of the plurality of discrete entities (100, 1800) based on the first optical read-out. Identifying at least one discrete entity (100, 1800) of the plurality of discrete entities (100, 1800) from the imaging container (102) as a discrete entity (100, 1800) of interest based on the at least one characteristic related to the biological sample (108) and the first representation of the marker (110) of said at least one discrete entity (100, 1800).

## Description

### Technical field

The invention relates to a method for assaying a plurality of biological samples. The invention further relates to a device for assaying a plurality of biological samples.

### Background

Rare cells, for example adult stem cells, circulating tumor cells and reactive immune cells (for example T-Cells, B-Cells, or NK-cells reactive to a certain antigen), are of great interest to fundamental and translational researchers. B-cell clones for example, that react to a certain pathogen, like a virus, and can produce antibodies against a particular target molecule of said pathogen are of great value to generate urgently needed therapeutic antibodies. Similarly, reactive T-cells are sought after in the context of personalized medicine and the treatment of cancer and other diseases. Once a reactive T-cell is identified and isolated, the genetic sequence encoding the corresponding T-cell receptor displaying affinity against the target antigen can be cloned and used to generate genetically engineered T-cells such as CAR-T cells. Similarly, circulating tumor cells are expected to have great value for diagnosing cancer, predicting outcomes, managing therapies, and for the discovery of new cancer drugs and cell-based therapeutics. Suspensions of cells containing rare cells are typically derived from either a tissue sample by means of dissociation or from a liquid biopsy. The identification, analysis, and isolation of rare cells in these samples, particularly the analysis of these cells on the single cell level (single cell analysis, SCA) is therefore of great value for basic and translational research, diagnostic and therapeutic applications as well as in the context of bioprocessing and development and manufacturing of biologics and cellular therapeutics.

Recent progress in the fields of cell culture research has led to the advent of 3D cell culture, which is based on cultivating cells in three dimensions, for example, in suspension culture (scaffold-free techniques) or embedded in hydrogels and/or extracellular matrices (scaffold-based techniques). Hydrogels and extracellular matrices have been used extensively in conjunction with other elements for scaffold-based 3D cell culture. Cells and other elements can be efficiently embedded into discrete entities such as for example hydrogel beads by various means, cultivated in suspension, and imaged. Various forms of hydrogel beads including single-phase, multi-phase, mixed phase, hollow as well as solid core hydrogel beads with or without a shell can be manufactured using a variety of approaches including, microfluidics, 3D printing, emulsification or electro-spraying.

This allows cultivation of large numbers of cells, including rare cells, for analytical, diagnostic and therapeutic purposes in a 3D cell culture.

The cultivation of cells embedded in hydrogels (i.e. scaffold-based cell culture), which are kept in suspension, combines the benefits of scaffold-based cell culture with the benefits of suspension cell culture and is thus an attractive mode of cell culture for a wide range of applications. For many workflows that could be based on this type of cell culture, it would be required to be able to repeatably recognise individual discrete entities. For example, in case of handling a large number of single cells, including rare cells, in a collective 3D cell culture, it is of great interest to be able to follow individual entities or cells, for example, over the course of an experiment. This allows identification, analysis, and isolation especially of rare cells within the large number of cells. However, handling large numbers of entities and cells in a single vessel, whilst keeping track of each, is currently not possible.

### Summary

It is an object to provide a method and a device for assaying a plurality of biological samples that allows a user to uniquely identify and keep track of individual biological samples which have been identified based on at least one characteristic related to the biological samples.

The afore-mentioned object is achieved by the subject-matter of the independent claims. Advantageous embodiments are defined in the dependent claims and the following description.

The proposed method for assaying a plurality of biological samples comprises the following steps: Preparing a plurality of discrete entities, each discrete entity comprising a polymeric compound, at least one of the biological samples and a marker. Arranging the plurality of discrete entities in an imaging container. Generating at least one first optical read-out of at least one discrete entity of the plurality of discrete entities. Determining a first representation of the marker and at least one characteristic related to the biological sample for at least one discrete entity of the plurality of discrete entities based on the first optical read-out. Identifying at least one discrete entity of the plurality of discrete entities from the imaging container as a discrete entity of interest based on the at least one characteristic related to the biological sample and the first representation of the marker of said at least one discrete entity.

The plurality of discrete entities is prepared outside the imaging container, for example by forming hydrogel beads comprising at least one of the biological samples and a marker each. The biological sample may in particular be any of the biological samples mentioned above, for example reactive immune cells, reactive T-cells or tumorous cells. The markers are optically detectable patterns, structures and/or distributions of optically detectable elements. Specific markers are described below with reference to embodiments. During the preparation of the plurality of discrete entities further discrete entities may be produced by accident which comprise no biological sample and/or no marker. These further discrete entities are not considered part of the plurality of discrete entities in this document. The prepared plurality of discrete entities is transferred or loaded into the imaging container, which may for example be a petri-dish, a microscope slide, a microwell plate or a similarly suited vessel. Then, the first optical read-out is generated from the imaging container comprising the plurality of discrete entities. The optical read-out may in particular be an image or a digital image. When the optical read-out is acquired, image data is generated based on the first optical read-out that is further processed to determine the first representation of the marker and the at least one characteristic related to the biological sample. Based on the first representation of the marker and the at least one characteristic related to the biological sample, at least one of the discrete entities is identified as one of the discrete entities of interest, for example for further processing. Further processing may include retrieval of the biological sample from the discrete entity of interest, further cultivation and expansion, biologics production, bioprocessing, fermentation, cloning of genes, and downstream analysis. Downstream analysis may include the genomic, transcriptomic, proteomic profiling, the analysis of transcription factor binding e.g. ChlP-Seq., DNA methylation profiling, metabolome analysis, amongst others.

When a large number of biological samples is handled, it is hard to keep track of individual samples, in particular, when the large number of biological samples is handled in a single vessel such as the imaging container. The discrete entities combine one of the biological samples with a marker each. Because the marker is configured to be unique for the biological sample, it therefore allows a user to uniquely identify each biological sample. Thus, the method allows the user to keep track of biological samples pooled in the imaging container over the course of an experiment. In particular, the method allows the user to uniquely identify and keep track of the discrete entities of interest which have been identified based on the at least one characteristic related to the biological sample. Thus, the method enables running time-lapse experiments with single cells, groups of cells, co-cultures, spheroids, tumoroids, or organoids, for example, cell-based assays including cell viability assays, cell proliferation assays, cytotoxicity assays, cell senescence assays, and cell death assays.

Alternatively, the proposed method for assaying a plurality of biological samples, comprises the following steps: Preparing a plurality of discrete entity precursors, each precursor comprising a polymeric compound and at least one of the biological samples enclosed by the polymeric compound. Arranging the plurality of discrete entity precursors in an imaging container. Creating a plurality of discrete entities from the plurality of discrete entity precursors, each discrete entity comprising the polymeric compound, the biological samples and a marker. Generating at least one first optical read-out of at least one discrete entity of the plurality of discrete entities. Determining a first representation of the marker and at least one characteristic related to the biological sample for at least one discrete entity of the plurality of discrete entities based on the first optical read-out. Identifying at least one discrete entity of the plurality of discrete entities from the imaging container as a discrete entity of interest based on the at least one characteristic related to the biological sample and the first representation of the marker of said at least one discrete entity.

The alternative method is distinguished from the method described above in that the plurality of discrete entities is prepared inside the imaging container from the plurality of discrete entity precursors. Otherwise the alternative method is identical to the method described above and has the same advantages. In particular, the alternative method can be supplemented with the features described in connection with the method described above.

In a preferred embodiment, the biological sample and the marker are enclosed by the polymeric compound. Alternatively or additionally, the polymeric compound comprises a marker and the biological sample - with or without a marker marking the biological sample - is enclosed by the polymeric compound. The polymeric compound may be polymerized when preparing the discrete entities in order to form the discrete entities. Polymerizing the polymeric compound enables culturing of the biological samples in the discrete entity, for example in a scaffold-based suspension 3D cell culture, which combines the benefits of 3D suspension cell culture and scaffold-based cell culture. In particular, it provides a way to leverage key benefits of 3D suspension cell culture including the improvement of mixing and aeration by use of agitated vessels, such as rotating bioreactors, spinner flasks, stirred tanks or rocking bed bioreactors, the possibility of running a continuous medium exchange, and the possibility to perform inline process control, while growing samples in a scaffold, i.e. a hydrogel, extracellular matrix and/or basement membrane, which provides a much more physiologically relevant environment for the samples with respect to matrix elasticity, chemical composition, attachment sites, and para- and autocrine signaling.

In another preferred embodiment, the method comprises the additional step of immobilizing the plurality of discrete entities or the plurality of discrete entity precursors in the imaging container. The discrete entities may be immobilized by attaching the discrete entities to the imaging container, for example by centrifuging the discrete entities to a surface of the imaging container. Immobilizing the plurality of discrete entities prevents the discrete entities from moving in the imaging container when the imaging container is moved, for example during the generation of an optical readout, thereby making it easier to keep track of the discrete entities of interest.

A substantially even distribution of the discrete entities in the imaging container may be achieved easily by centrifuging the discrete entities to a surface of the imaging container. This may also serve to immobilize the discrete entities either by attaching them to a functionalized surface or by embedding them into a second hydrogel or polymer. Such an embedding may be achieved by either placing discrete entities into a hydrogel or polymer prior or concomitant to polymerization of said hydrogel or polymer or by placing the discrete entities first followed by overlaying them with a suitable hydrogel or polymer. Said hydrogel or polymer may be the same that is used for preparation of the discrete entities with a different viscosity, i.e. gel concentration. For example, one may prepare the discrete entities using 0.8% low-melting agarose and embedding them into a 0.6% low-melting agarose. Alternatively, the matrix used for the preparation of the discrete entities may be different from the one used for the embedding of the discrete entities. The matrix or polymer used for embedding the discrete entities may contain functionalizations that allow the selective depolymerization by either UV light-induced, temperature-induced, chemically-induced or enzymatically-induced cleavage. This may serve to allow the controlled release of the discrete entities of interest.

In another preferred embodiment, the method comprises the additional step of extracting at least one discrete entity of the plurality of discrete entities from the imaging container based on the at least one characteristic related to the biological sample and the first representation of the marker of said at least one discrete entity. In this embodiment, at least one discrete entity of interest is removed from the imaging container for further processing. For example, the discrete entity may be removed from the imaging container by means of aspiration, i.e. suction. Removing the discrete entity of interest from the imaging container allows for example for further study in a specific environment away from the remaining discrete entities.

In another preferred embodiment, the method comprises the additional steps of: Generating at least one second optical read-out after a predefined period of time has passed. Determining a second representation of the marker and the characteristic related to the biological sample for at least one discrete entity of the plurality of discrete entities based on the second optical read-out. Comparing the first representations and the second representations in order to identify at least one discrete entity of the plurality of discrete entities. Comparing the characteristic related to the biological sample determined from the first optical read-out to the characteristic related to the biological sample determined from the second optical read-out. In this embodiment, the method preferably comprises the additional step of extracting at least one discrete entity of the plurality of discrete entities from the imaging container based on said comparison. In this embodiment, a time series measurement is performed. At the first time point of the series, the first optical read-out is generated and a first value of the characteristic related to the biological sample is determined. At the second time point of the series, the second optical read-out is generated and a second value of the characteristic related to the biological sample is determined. Between the first optical read-out and the second optical read-out, the value of the characteristic may have changed. For example, from the second optical read-out a specific protein expression may be determined.

In another preferred embodiment, the method comprises the additional step of cultivating the discrete entities, in particular the biological samples being part of the discrete entities, between the first optical read-out and the second optical read-out. Cultivating the biological sample may include treatment with a chemical compound, a biologic compound (for example a protein, a signaling molecule, a nucleic acid, an antigen, a neoantigen, an antibody), a pathogen (for example a virus, a bacterium, a parasite), a biologically active agent (for example CRISPR, RNAi), a mechanical, electrical, or photophysical stimulus. Accordingly, this embodiment enables workflows in which the state of the biological sample is compared between at least two time-points, i.e. before and after the cultivation step. The state of the biological samples before and after the treatment is determined from the first optical read-out and the second optical read-out. Further, this embodiment enables time-lapse experiments, for example with single cells, groups of cells, co-cultures, spheroids, tumoroids or organoids as the biological sample. Likewise, a variety of cell-based assays commonly used in phenotypic screening can be performed in this embodiment. Such cell-based assays include cell viability assays, cell proliferation assays, cytotoxicity assays, cell senescence assays, and cell death assays. Importantly, cells or groups of cells that have been identified as biological sample of interest in this way, can be extracted and/or subjected to further processing.

In another preferred embodiment, the method comprises the additional step of arranging a fluid or liquid medium inside the imaging container before arranging the plurality of discrete entities or the plurality of discrete entity precursors inside the imaging container. The liquid medium allows the plurality of discrete entities or the plurality of discrete entity precursors to be suspended inside the imaging container, making the pluralities easier to handle. Further, the fluid or liquid medium may be biologically active, facilitating the cultivation of the biological samples. The liquid medium may comprise cell culture media, for example DMEM, buffers, for example PBS, PBDT, or synthetic hydrogels. The liquid medium may also comprise complex mixtures, for example naturally derived mixtures such as JellagelTM, Matrigel, and Collagen.

In another preferred embodiment, the method comprises the additional step of arranging a protective layer atop the liquid medium. The protective layer protects the liquid medium and the discrete entities from drying out and against environmental influences. The protective layer may be formed from any substance mentioned above with reference to the liquid medium. More than one protective layer may be formed. The protective layer and the liquid medium may be of the same substance having different properties such as density and viscosity. The difference between protective layer and the liquid medium may in particular be the concentration, for example a 0.6% low-melting agarose will be extremely soft, a 2% low-melting agarose relatively stiff. The elastic modulus of biological tissues varies greatly from bone matrix, cartilage, connective tissue to endometrium, for example. Using biologically inspired compositions that contain some key extracellular matrix components specific to these tissues is a way to generate various materials that have excellent biocompatibility. Examples of such proteins are Thrombospondin, Tenascin, COMP, Collagens, Collagen IV, further, there are hyaluronans and glycosylation groups that may be used. Bioinks used to 3D print in the area of tissue engineering are another source of gels with good biocompatibility that may be used either as the liquid medium or the protective layer.

In another preferred embodiment, the method comprises the additional step of adding a reagent to the liquid medium in order to form a gel or a polymeric compound enclosing the plurality of discrete entities or the plurality of discrete entity precursors. By forming the gel or the polymeric compound, the plurality of discrete entities may be immobilized, preventing individual discrete entities from moving, for example when the imaging container is handled. Matrices for enclosing the plurality of discrete entities or the plurality of discrete entity precursors may be formed by functionalization and/or cross-linking using various cross-linking agents that can be added to the liquid medium or via using reactive groups on monomers.

In another preferred embodiment, the method comprises the additional step of providing a compound, in particular a biological compound, that is sensitive to the characteristic related to the biological sample in the imaging container. The characteristic related to the biological sample may be determined based on a reaction of the compound. The compound may for example comprise specialized reporter cells, FirePlex particles, or reporter arrays that are configured to capture analytes of interest such as secreted proteins to render them detectable using appropriate detection and labeling reagents. Preferably, the compound is provided as a layer, in particular a monolayer, on the bottom of the imaging container. Thus, the method may comprise the additional step of preparing the layer on the bottom of the imaging container, in particular before the plurality of discrete entities or the plurality of discrete entity precursors is arranged in the imaging container. Providing the compound as part of the discrete entities themselves may result in the compound being unevenly distributed among the discrete entities, and thus the biological samples. Contrary thereto, providing the compound in form of the layer ensures that the compound is evenly distributed around the biological samples. In an example, the biological samples are clones of cells and a desired characteristic related to the biological sample is the secretion of a particular monoclonal antibody (mAb). The compound is configured to react to the presence of the particular monoclonal antibody, for example by comprising a native antigen. In particular, the compound is configured to react to the presence of the particular monoclonal antibody in a way that is optically detectable. For example, the presence of the particular monoclonal antibody may change an optical property of the compound such as transparency, color or the fluorescence signal, for example brightness, spectral change, fluorescence lifetime. The change of the optical property may be localized to where the respective biological sample of interest is located. Thus, the presence of the particular monoclonal antibody can be detected from the first optical read-out and/or the second optical read-out. Once the presence of the particular monoclonal antibody has been detected, the discrete entities comprising the biological sample secreting said monoclonal antibody may be labeled as the discrete entities of interest. The discrete entities of interest may be extracted or otherwise further processed.

In another preferred embodiment, each marker comprises at least one of a luminescent microbead, a non-fluorescent microbead, a luminescent nanoruler, and a dye. The microbead and the nanoruler may be fluorescent or phosphorescent. If the marker comprises fluorescent or non-fluorescent microbeads, it is possible to use different kinds of microbeads, these different kinds of microbeads can include microbeads of different sizes (diameters), different shapes, different (fluorescent) emission characteristics, such as different emission spectra or different fluorescent lifetimes. The marker may be distributed on the surface of the discrete entity, embedded in the polymeric compound, attached to the biological sample and/or embedded in the biological sample. In this embodiment, a fast, non-destructive and contactless detection of the marker is possible. Preferably, the markers are adapted in a way which enables a unique identification of the at least one discrete entity of the plurality of discrete entities, for example by randomly arranged but statically distributed luminescent microbeads in a three-dimensional manner in or at the discrete entity, as explained in further detail below. The aforementioned elements of the marker can be easily read-out at very high acquisition speeds and lend themselves well for segmentation or feature extraction. Further, the elements of the marker can be easily removed from the image data by means of image processing. This is advantageous as the marker might otherwise obstruct the view of the included biological specimen.

In another preferred embodiment, each marker comprises at least one element, which has a directional component, in particular a nanoruler, a rod-shaped nanostructure or an elongated microbead. The directional component may also be any other suitable object with a single central axis, such as a longitudinal axis, and with the orientation of the object identifiable by the single central axis.

In another preferred embodiment, each marker comprises a three-dimensional pattern. The marker may in particular be generated by means of lithography, in particular by photolithography, by multi-photon photolithography or by 2-photon lithography. The three-dimensional pattern may be generated after the discrete entity is formed. Using multi-photon lithography, three-dimensional patterns can be generated comparable to stacked barcodes. Such three-dimensional patterns can hold a significantly higher information content compared to two-dimensional codes, and allow a large number of unique codes to be generated. The three-dimensional patterns may also enable the storage of additional information relating to the sample origin, the experiment, and/or user in the pattern, and therefore directly in the discrete entity. It is important to note that such three-dimensional patterns are substantially stable over a long period of time and may provide significant advantages not only in the context of the workflows described in this document, but also may prove very valuable for banking of biological samples.

The invention also relates to a device for assaying a large number of biological samples, configured to perform the method according to any one of the previous claims. The device comprises an imaging container configured to receive the plurality of discrete entities and/or the plurality of discrete entity precursors, and an optical read-out unit configured to perform at least one optical read-out of the plurality of discrete entities.

A control unit is configured to at least determine a first representation of the marker and at least one characteristic related to the biological sample for each discrete entity of the plurality of discrete entities based on the at least one optical read-out performed by the optical read-out unit. In particular, the control unit is a part of the device. Alternatively, the control unit may be arranged remotely from the device. The remotely arranged control unit may be connected to the rest of the device via a computer network.

The device has the same advantages as the claimed methods. In particular, the device can be supplemented with the features of the dependent claims directed to the methods. Furthermore, the methods described above can be supplemented with the features described in this document in connection with the device.

The invention also relates to a computer program product comprising a program code that causes the device described above to perform one of the methods described above when run on a processor.

The computer program product has the same advantages as the methods and the device described above. In particular, the computer program product can be supplemented with the features of the dependent claims directed to the methods.

The invention further relates to a computer-readable medium comprising the computer program described above.

### Short Description of the Figures

Hereinafter, specific embodiments are described referring to the drawings, wherein:
- Figures 1 and 2: are schematic views of a plurality of discrete entities arranged in an imaging container;
- Figure 3: is a schematic view of the steps of the method for assaying the plurality of biological samples according to an embodiment;
- Figure 4: is a schematic view of the steps of the method for assaying the plurality of biological samples according to another embodiment;
- Figures 5 to 8: are schematic views of the steps of the method for assaying the plurality of biological samples according to another embodiment;
- Figures 9 to 12: are schematic views of the steps of the method for assaying the plurality of biological samples according to another embodiment;
- Figures 13 to 17: are schematic views of the steps of the method for assaying the plurality of biological samples according to another embodiment;
- Figures 18 and 19: are schematic views of the steps of the method for assaying the plurality of biological samples according to another embodiment;
- Figure 20: is a schematic views of a step of the method for assaying the plurality of biological samples according to another embodiment; and
- Figure 21: is a schematic view of a device for assaying a large number of biological samples.

### Detailed Description of the Figures

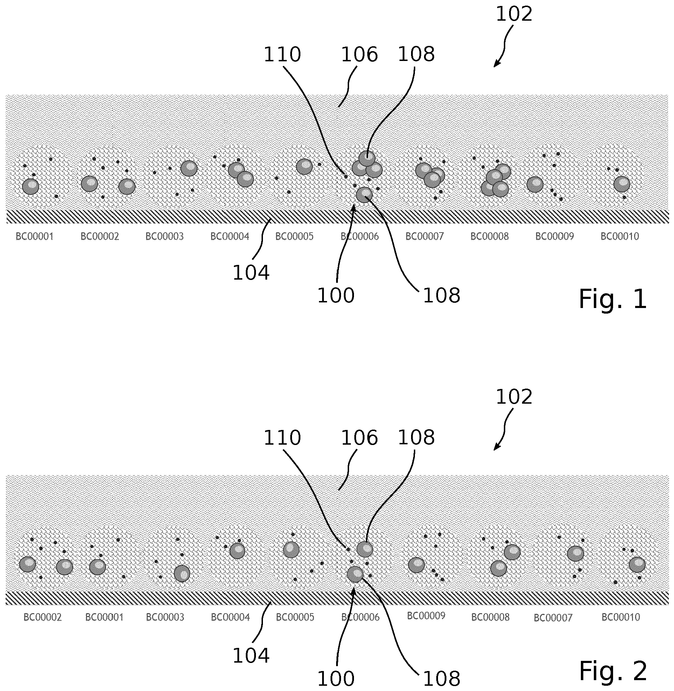

Figures 1 and 2 are schematic views of a plurality of discrete entities 100 arranged in an imaging container 102.

The views of Figure 1 and 2 each show a schematic cross-section through the imaging container 102 which may be a petri-dish, a microscope slide, a microwell plate or a similarly suited vessel. The plurality of discrete entities 100 is exemplary arranged in a monolayer on a bottom surface 104 of the imaging container 102. In order to protect the discrete entities 100 and to simplify handling, the imaging container 102 comprises a liquid medium 106 in which the discrete entities 100 are suspended. This liquid medium 106 may be water, a cell growth medium, an oil or any other suitable liquid.

The discrete entities 100 are exemplary formed as beads and comprise at least one polymeric compound, in particular, a polymeric compound that forms a hydrogel and/or that is substantially optically transparent. Accordingly, the beads are also called hydrogel beads in this document. The polymeric compound may be of natural or synthetic origin, including agarose, alginate, chitosan, hyaluronan, dextran, collagen and fibrin as well as polyethylene glycol), poly(hydroxyethyl methacrylate), poly(vinyl alcohol) and poly(caprolactone). Further examples include basement membrane extracts, which may include Laminin I, Collagen I, Collagen IV, Vitronectin and Fibronectin amongst others, and extracellular matrix preparations, including for example, Cultrex, Matrigel, or Jellagel.

The discrete entities 100 may comprise a single or several different polymeric compounds. In particular, the discrete entities 100 may comprise several sections, for example as an inner core and an outer layer or outer shell around the inner core. Each of the sections can be made of a particular polymeric compound. In addition, the discrete entities 100 may comprise sections that are made of other compounds that do not form hydrogels. Thus, the sections of the discrete entities 100 can each have different properties. These properties include physicochemical properties such as Young's modulus, refractive index, and chemical composition and functionalization.

The shape of the discrete entities 100 exemplary shown as spherical. Alternatively, the discrete entities 100 have a different shape, for example a spheroid shape. The diameter of the discrete entities 100 may be in the range of 10 µm to 10 mm. Particularly preferred ranges are 10 µm to 100 µm, 50 µm to 250 µm and 500 µm to 5 mm.

Each discrete entity 100 comprises at least one biological sample 108 enclosed by the hydrogel bead formed from the polymeric compound. The biological samples 108 are exemplary shown as a cluster of cells in Figures 1 and 2. The cells may be eukaryotic or a prokaryotic cells, including archaea, bacteria, plant, mammalian, non-mammalian cells and fungi. The cluster of cells may be a spheroid, a tumoroid or an organoid. In addition, the biological sample 108 can be a co-culture of different cell types, bacteria, viruses, prions, cellular pathogens and/or multicellular parasites. The biological sample 108 can be suitable for analytical, diagnostic, and/or therapeutic applications.

The discrete entities 100 shown further comprise a marker 110. The markers 110 shown in in Figures 1 and 2 are exemplary formed by a plurality of fluorescent microbeads. The fluorescent microbeads are included and randomly dispersed in the polymeric compound, preferably during the formation of the discrete entity 100. After the formation of the discrete entity 100, the microbeads are set in place in the discrete entity 100. This means the microbeads do not change their location in the discrete entity 100 once the discrete entity 100 is formed. The discrete entity 100 might be deformed at a later point in time, for example by growth of the cells, an influence caused by cultivation or processing of the discrete entity 100 in the liquid. The diameter of the microbeads is in the range of 50nm to 500nm.

The constituent parts of the markers 110 may also be structures or compounds that are different from the fluorescent microbeads, for example non-fluorescent microbeads, areas of the discrete entity 100 that are colored by means of a dye, or a continuous distribution of phase or intensity objects. The markers 110 or parts thereof may also derive from the biological sample 108.

In an example, the constituent parts of the markers 110 may include structures of the hydrogel beads that are generated during formation of the discrete entities 100, such as random imperfections on a surface of the hydrogel beads and that are inherent to the method of formation of the hydrogel beads. In this case, the constituent parts of the markers 110 may be on the outside, particularly on the surface, of the hydrogel beads. Further constituent parts of the markers 110 may be applied to the surface of the hydrogel beads such that the entirety of the markers 110 or parts thereof are on the surface of the hydrogel beads. Additionally, the markers 110 may be applied to the surface of an internal phase of multi-phase hydrogel beads such that the markers 110 will form a spherical layer inside of the hydrogel beads of the discrete entities 100. Further, the constituent parts of the markers 110 can be a color gradient or an intensity gradient.

In another example, the markers 110 comprise nanorulers which are at least two intensity or phase point objects physically connected in a rigid manner. For example, the nanorulers may be composed of a nucleic acid and a series of dyes that each have different spectral and/or life time properties. The dyes are coupled to the nucleic acid at a predetermined distance. Nanorulers and similar structures are constituent parts of the markers 110 and may be used to generate the markers 110 instead of or in addition to e.g. microbeads. As the nanorulers have an intrinsic directionality or shape, they can be directly represented as a vector or as a respective shape when generating representations of the markers 110. As each of these vectors can point into any direction they considerably increase dynamic range and the number of unique markers 110. Even for an identical number of nanorulers in one of the hydrogel beads of the discrete entities 100 and an identical placement of the nanorulers with respect to the X,Y,Z position of their centres of mass of said hydrogel bead, the rotational degrees of freedom result in a high number of possible orientations of the same, and therefore in a high number of unique representations of the markers 110. Something comparable can be accomplished by using microbeads having an asymmetric or elongated shape or comparable fluorescent particles.

In another example, the constituent parts of the markers 110 may be generated after the formation of the hydrogel beads of the discrete entities 100. This can be achieved by including compounds in the hydrogel beads when forming the discrete entities 100, the compounds forming the constituent parts of the marker 110. Compounds included in the hydrogel beads may be activated, deactivated or bleached photochemically after formation of the discrete entity 100. In a subsequent lithographic, in particular in a photolithographic step, the compounds may be activated, deactivated or bleached photochemically by means of a focused light beam, or by imaging or projecting a pattern on the discrete entity 100.

In further example, the constituent parts of the markers 110 may be generated photochemically or photophysically in a stochastic or deterministic manner. When generated stochastically, changed areas are generated at random locations in the hydrogel beads of the discrete entities 100. For example, random distributions of stripes, points, or similar feature distributions may be randomly generated. The photolithographically generated markers 110 may be in the form of barcodes or stacked barcodes. A multi-photon photolithographic device 2100 may be used to generate photochemically or photophysically changed areas that are arranged in a three-dimensional pattern in the hydrogel bead of the discrete entity 100.

When generated deterministically, the changed areas are generated such that for each hydrogel bead of the discrete entities 100, a unique pattern of changed areas is generated. In particular, this may be in the form of barcodes. Further, this unique pattern may encode additional information, such as a unique identifier as well as further information including information about the origin of the sample or biopsy embedded within the discrete entity 100, the treatment of the sample, date, time, experimenter. Importantly, using multi-photon lithography it is possible to write three dimensional codes of this type, which significantly increases bit depth of the encoding and thus the amount of information that may be encoded with the unique pattern.

Photolithographically generated patterns of photochemically or photophysically changed areas of the discrete entities 100 may be either positives or negatives. Negatives, for example, may be generated by uncaging or deprotecting protected binding sites photochemically, which generates deprotected binding sites in the respective areas. Negatives may then be developed into positives by coupling dyes covalently using click chemistries to unprotected reactive sites for example, which can be efficiently performed by bathing hydrogel beads in activated dye solution followed by washing out unbound dye molecules. As the protected binding sites can be covalently linked to the hydrogel polymer, covalently coupled dye molecules or other covalently coupled constituent parts of the marker 110 establish a substantially stationary pattern or distribution. By generating a unique pattern or a unique distribution of these specific locations, the marker 110 is generated either directly (direct generation of the positive) or indirectly (direct generation of the negative followed by development which leads to the positive). In any case, the constituent parts of the marker 110 are optically detectable, for example, as a phase or intensity object by means of a microscope. Thus, the marker 110 is optically detectable. The hydrogel beads of the discrete entities 100 are required to be transparent, at least to an extent that allows the biological samples 108 and the markers 110 to be optically detectable.

The discrete entities 100 may be prepared outside the imaging container 102 and then be transferred to the imaging container 102. The discrete entities 100 may be formed by electro spraying, emulsification, lithography, and 3D printing. The formation of the discrete entities 100 may be performed in a dedicated culture vessel such as a spinner flask or a microwell plate. In particular, the discrete entities 100 are formed by means of a microfluidic device which may be a part of the device 2100 for assaying a large number of biological samples 108. Alternatively, a plurality of discrete entity precursors is arranged in the imaging container 102 and the plurality of discrete entities 100 is formed inside the imaging container 102 itself.

The view of Figure 1 depicts one step in a method for assaying a plurality of biological samples 108. The plurality of biological samples 108 to be assayed is formed by the biological samples 108 encapsulated in the discrete entities 100. Each of the biological samples 108 may be uniquely identified by the marker 110 that is part of the same discrete entity 100 as said biological sample 108. In Figure 1, each of the plurality of discrete entities 100 is labeled by an identifier BC00001 to BC00010.

The step depicted in Figure 1 comprises generating a first optical read-out of the plurality of discrete entities 100, for example by imaging the plurality of discrete entities 100 or parts thereof with a microscope. The optical read-out may in particular be an image of the plurality of discrete entities 100 or parts thereof, respectively. From the first optical read-out first data is generated that is further processed to generate first representations of all the markers 110 of the discrete entities 100 contained in the first read-out. Further, at least one characteristic related to the plurality of biological samples 108 is determined from the first data corresponding to the first optical read-out. This characteristic may, for example, be the presence of a certain bio-marker 110. A further step of the method is described in the following with reference to Figure 2.

The view of Figure 2 depicts another step in the method for assaying a plurality of biological samples 108. The step depicted in Figure 2 comprises generating a second optical read-out of the plurality of discrete entities 100 after the discrete entities 100, in particular the biological samples 108 being part of the discrete entities 100, have been cultivated. Cultivation may, for example, include treating the biological samples 108 with a compound or otherwise stimulating the biological samples 108. The second optical read-out is performed similar to the first optical read-out, and second data is generated from the second optical read-out. The second data is further processed to determine second representations of all the markers 110 of the discrete entities 100 contained in the second read-out, and the at least one characteristic related to the plurality of biological samples 108.

As can be seen from the labels in Figure 2, the individual discrete entities 100 have been rearranged between the step depicted in Figure 1 and the step depicted in Figure 2. The rearrangement may have been caused by moving the imaging container 102 or other distortions. Since each discrete entity 100 is uniquely labeled by one of the markers 110, it is possible to track individual discrete entities 100 between steps. This allows a user to track the reaction of individual biological samples 108 to the cultivation based on the characteristic determined from the second optical read-out by comparing it to the characteristic determined from the first optical read-out.

Figures 1 and 2 serve to illustrate the use of the discrete entities 100 combining one of the biological samples 108 with a unique marker 110 each inside a hydrogel bead in the method for assaying a plurality of biological samples 108. The features, in particular the features of the discrete entities 100, described above may be implemented in all embodiments of the method. In the following, the method will be described in more detail with reference to various embodiments. In this document, when referring to the discrete entities 100 used in the method, the terms plurality of discrete entities 100 and the discrete entities 100 may be used interchangeably.

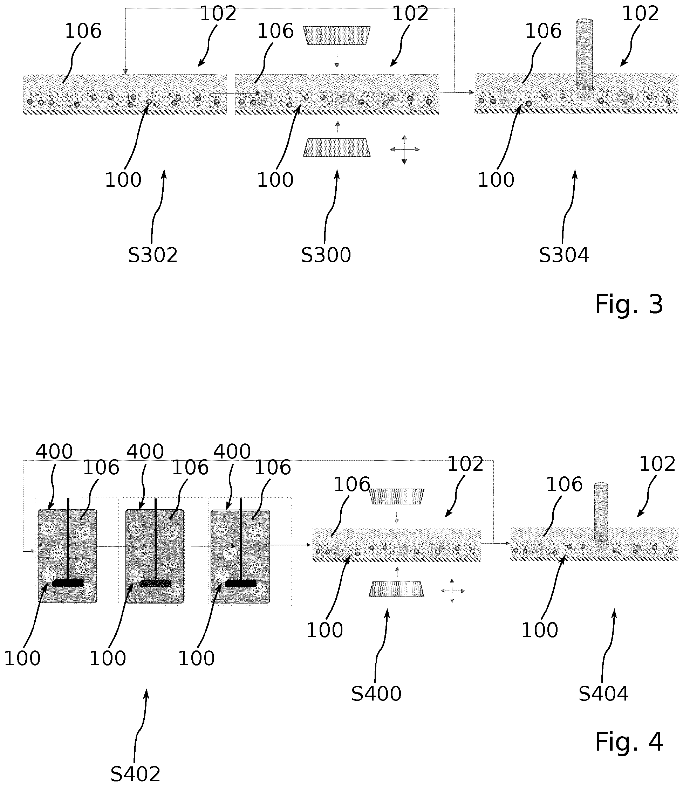

Figure 3 is a schematic view of the steps of the method for assaying the plurality of biological samples 108 according to an embodiment.

The discrete entities 100 are formed either outside the imaging container 102 or inside the imaging container 102 by encapsulating one of the plurality of biological samples 108 in one of the hydrogel beads, respectively. Additionally, one of the markers 110 is generated in one of the discrete entities 100, respectively. The markers 110 may be encapsulated in the same step as the biological samples 108 and/or the markers 110 may be generated after the hydrogel beads encapsulating the biological sample 108 has been formed. Like the encapsulation of the biological samples 108, the generation of the markers may be performed outside the imaging container 102 or inside the imaging container 102. When the generation of the markers is performed inside the imaging container 102, the hydrogel beads comprising only the biological samples 108 are also referred to as discrete entity precursors.

After the plurality of discrete entities 100 has been formed and/or arranged inside the imaging container 102, the first optical read-out is performed in step S300. The first data generated from the first optical read-out is analyzed. For each discrete entity 100 that has been captured by the first optical read-out, a first representation of the marker 110 of said discrete entity 100 is generated. This first representation allows to keep track of the respective discrete entity 100 in future read-outs. Likewise, from the first data the at least one characteristic related to the biological sample 108 is determined. The first representation and specific value of the characteristic may be stored for each discrete entity 100 that has been captured by the first optical read-out. Additionally, the first data corresponding to the first optical read-out may be stored.

In step S302, the plurality of discrete entities 100 is cultivated inside the imaging container 102. This cultivation step may comprise various treatments with reagents, for example chemical compounds, biologics, DNA, RNA, proteins, antibodies, antibody-fragments, aptamers, or physical stimuli, for example mechanical, electrical, photophysical stimuli, wounding, scratching, ablation, photoablation, temperature changes, changes to physiological conditions (pH, O₂, CO₂, N₂ concentrations). The cultivation step may also include the addition of further cell types, bacteria, archea, fungi, multicellular eukaryotes, parasites, animals, and plants. The cultivation step may in particular include letting the biological samples 108 rest for a predetermined amount of time.

After the cultivation step, step S300 is repeated for the second optical read-out. From the second data generated from the second optical read-out, the second representation is generated for each marker 110 of each discrete entity 100 that has been captured by the second optical read-out. The captured discrete entities 100 may then be identified based on the second representation. The at least one characteristic related to the biological sample 108 is determined as well from the second data. Using the identification based on the first representation and the second representation, the values of the at least one characteristic are compared for each biological sample 108 between the acquisition of the first optical read-out and the second optical read-out. The second representation and specific value of the characteristic may be stored for each discrete entity 100 that has been captured by the second optical read-out. The same may be done for further optical read-outs. Additionally, the first data corresponding to the first optical read-out may be stored.

The steps S300 and S302 may be repeated again for further optical read-outs to generate a number of sequential assays and/or a time-series. A number of discrete entities 100 may be identified as discrete entities of interest in this manner. In step S304, at least one of the discrete entities 100 that has been identified as one of the discrete entities of interest is removed from the imaging container 102 for further processing. This further processing may include retrieval of the biological sample 108 from the discrete entity 100, further cultivation and expansion, biologics production, bioprocessing, fermentation, cloning of genes, and downstream analysis. Downstream analysis may include the genomic, transcriptomic, proteomic profiling, the analysis of transcription factor binding e.g. ChlP-Seq., DNA methylation profiling, metabolome analysis, amongst others.

Figure 4 is a schematic view of the steps of the method for assaying the plurality of biological samples 108 according to another embodiment.

The embodiment of the method shown in Figure 4 is distinguished from the embodiment shown in Figure 3 in that the cultivation step is performed outside the imaging container 102. Between the steps S400, in which the optical read-outs are performed, the liquid medium 106 in which the discrete entities 100 are suspended is removed from the imaging container 102 and transferred into a separate vessel 400, exemplary shown as a stirring flask. In step S402, the plurality of discrete entities 100 is cultivated, stained, and/or washed in the separate vessel 400 before being transferred back into the imaging container 102 for performing another optical read-out. After the steps S400 and S402 have been repeated at least once, at least one of the discrete entities 100 that has been identified as one of the discrete entities of interest is removed from the imaging container 102 for further processing in step S404.

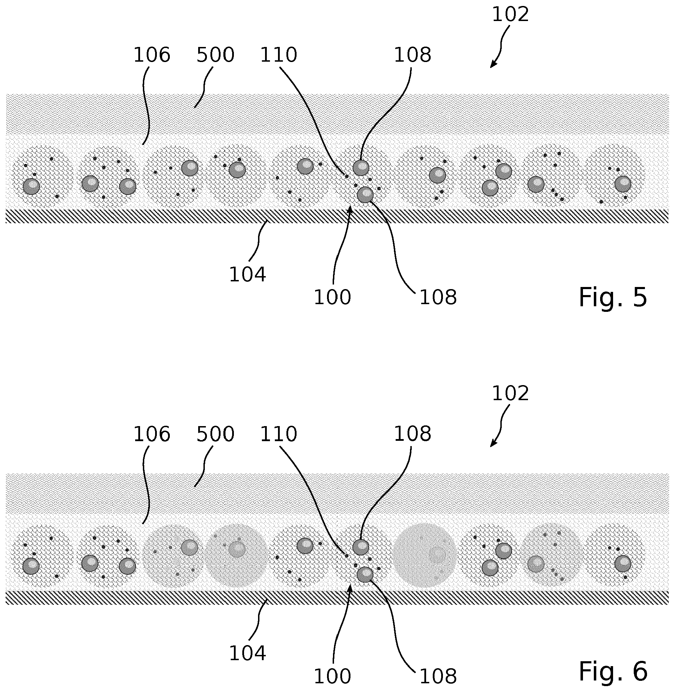

Figures 5 to 8 are schematic views of the steps of the method for assaying the plurality of biological samples 108 according to another embodiment.

In the present embodiment, the biological samples 108 enclosed are clonal cell that are assayed for the production of monoclonal antibodies (mAb). In addition to the biological samples 108, targets are encapsulated into the discrete entities 100. The targets may be target cells or target molecules and form a compound that is sensitive to the characteristic related to the biological sample 108 in this embodiment. The purpose of the method according to this embodiment is to find and extract the clonal cells that produce the monoclonal antibodies that best bind to the target.

The view of Figure 5 illustrates a first step of the method in which the plurality of discrete entities 100 is arranged in the liquid medium 106 inside the imaging container 102. A protective layer 500 is formed above the liquid medium 106 and the monolayer formed by the discrete entities 100. The protective layer 500 may be formed from the same substance as the liquid medium 106, for example having different density, viscosity, and/or concentration. The protective layer 500 prevents the liquid medium 106 from drying out and prevents spilling of the liquid medium 106 when the imaging container 102 is handled.

The view of Figure 6 illustrates a second step of the method in which an optical read-out of at least a part of the plurality of discrete entities 100 is generated. Data is generated from the optical read-out. The data is further processed, for example by means of a computer, to generate the representations of the markers 110 of the discrete entities 100 that have been captured by the optical read-out. Further, as characteristics related to the biological samples 108, i.e. the clonal cells, four quantities are determined. As a first characteristic it is determined how good the monoclonal antibodies produced by each of the clonal cells binds to the target. As a second characteristic a functionality of the monoclonal antibodies is determined. As a third characteristic the solubility of the monoclonal antibodies is determined. Finally, as a fourth characteristic the yield of monoclonal antibodies is determined for each of the clonal cells. To this end a fluorescently labeled secondary antibody reactive to the isotype of said monoclonal antibody is used to label the monoclonal antibody. The fluorescence intensity can then be used to readout how much monoclonal antibody is found in the discrete entity, its vicinity, on target cells expressing the antigen and/or on structures like microbeads or nanostructures that carry the antigen. The relationship between the amount of monoclonal antibody found in these compartments allows inference to be made on the aforementioned characteristics. Similarly, the presence of aggregates can be detected as fluorescent aggregates and allows inference to be made about the solubility of the antibody. More importantly, if reporter cells are being used that express the antigen two very important aspects can be assayed: First, to which extent a monoclonal antibody influences a biological function of the target cells. For example, two different monoclonal antibodies may be found that bind the same receptor tyrosine kinase with apparently similar affinity, but only one of them may inhibit signaling. This may be easily detected using reporter cells that bear a suitable genetic modification that reports the corresponding downstream signaling by expressing a fluorescent protein like mCherry, GFP, etc. or by a change of the FRET signal. Second, the binding to native epitope may assayed. Depending on the type of microscopic readout and label used these assays may deliver qualitative information or quantitative information. In case individual binding events between antibodies and targets are monitored over time actual binding strength, K_{D} values, ON-/OFF-rates can be determined. The same can be achieved, when fluorescence correlation spectroscopy is used in addition to the imaging readout. Such more elaborate and time-intensive measurements, may be performed as a secondary assay to qualify a small number of clones of interest in depth.

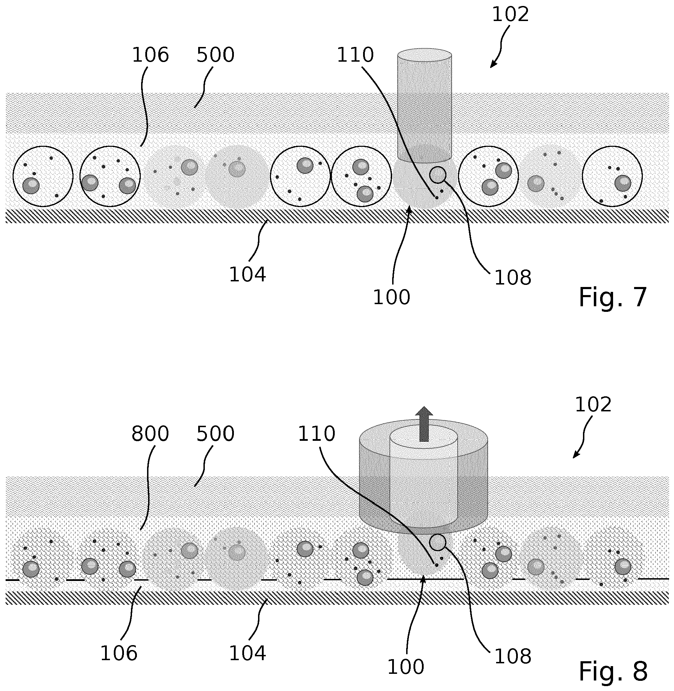

Based on the four characteristics, the most desirable clonal cells are determined. The discrete entities 100 comprising the most desirable clonal cells are labeled as the discrete entities of interest and extracted in a third step that is illustrated in the view of Figure 7. Exemplary, one of the discrete entities 100 is extracted comprising a clonal cell that produces a high yield of monoclonal antibodies that strongly bind to the target and display good solubility and function. The extracted discrete entities 100 may be identified during further processing based on the representation of the marker 110 that has been stored together with the determined characteristics for each of the discrete entities 100.

The view of Figure 8 illustrates an alternative method of extracting the desired clonal cells. In this alternative, a hydrolyzing agent 800 is introduced into the liquid medium 106 that dissolves the hydrogel beads of the discrete entities 100. After hydrolyzing the hydrogel beads, the biological samples 108, i.e. the clonal cells in this example, may be extracted from the liquid medium 106 directly, for example by aspiration. As hydrolyzing agent 800, a protease may be used, for example collagenase or dispase. In addition, or as an alternative to the hydrolyzing agent 800, a specific cleavage site and chemistry may be incorporated into the hydrogel or polymer used to generate the discrete entities. Such a cleavage group and chemistry may be a for example a cleavage group sensitive to a certain chemical, UV-light, temperature or particular enzyme, which may be then be used to dissolve the discrete entity easily. Such a cleavage group and chemistry may or may not be biorthogonal.

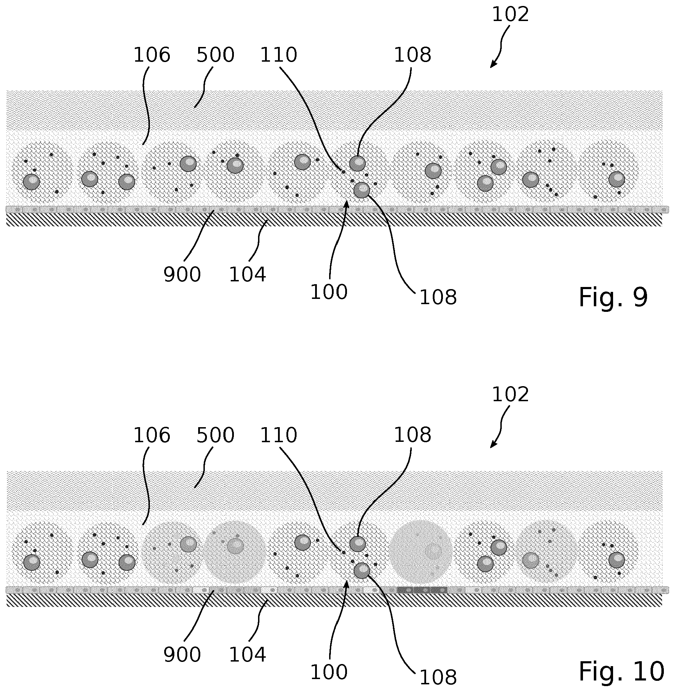

Figures 9 to 12 are schematic views of the steps of the method for assaying the plurality of biological samples 108 according to another embodiment.

As in the previously described embodiment, in the present embodiment, the biological samples 108 enclosed are clonal cells that are assayed for the production of monoclonal antibodies. However, in this embodiment, the targets are provided as a layer 900 on the bottom of the imaging container 102, for example a monolayer of cells comprising a native-antigen to the desired monoclonal antibody. The arrangement of the plurality of discrete entities 100 comprising the clonal cells and the monolayer of cells comprising the native-antigen is shown in the view of Figure 9, which corresponds to a first step in the method.

The view of Figure 9 illustrates a first step of the method in which the layer 900 comprising the target and the plurality of discrete entities 100 is arranged in the liquid medium 106 inside the imaging container 102. In this embodiment, like in the embodiment describe above, the target is compound that is sensitive to the characteristic related to the biological sample 108 being encapsulated in the discrete entities 100. In this embodiment, a protective layer 500 is formed as well above the liquid medium 106 and the monolayers formed by the discrete entities 100 and the targets.

The view of Figure 10 illustrates a second step of the method. The second step is performed after a predetermined amount of time has passed and the monoclonal antibodies produced by the clonal cells had time to diffuse out of the hydrogel beads of the discrete entities 100. Some of the monoclonal antibodies bind to the native antigen of the target in the layer 900 at the bottom of the imaging container 102. The amount of binding is indicated in Figures 10 to 12 by different hatching. Further, in the second step, an optical read-out of at least a part of the plurality of discrete entities 100 and/or at least a part of the layer 900 is generated. From the data corresponding to the optical read-out, the representations of the markers 110 are generated and may be analyzed and/or stored.

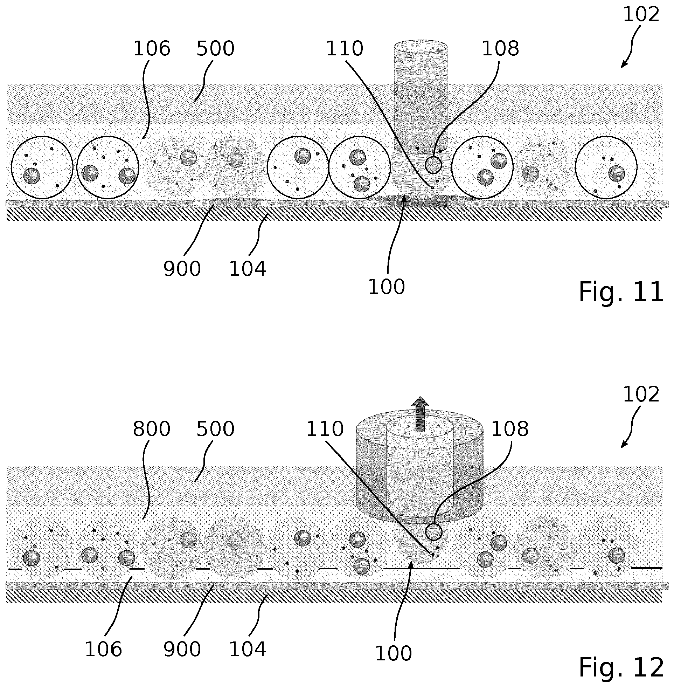

Further, it is determined how good the monoclonal antibodies produced by each of the clonal cells bind to the target, as well as the function and solubility of the monoclonal antibodies and the yield of each of the clonal cells. Based on these characteristics, the most desirable clonal cells are determined. The discrete entities 100 comprising the most desirable clonal cells are labeled as the discrete entities of interest and extracted in a third step that is illustrated in the view of Figure 11. The extracted discrete entities 100 may be identified during further processing based on the representation of the marker 110 that has been stored together with the determined characteristics for each of the discrete entities 100.

The view of Figure 12 illustrates an alternative method of extracting the desired clonal cells, in which the hydrolyzing agent 800 is introduced into the liquid medium 106 that dissolved the hydrogel beads of the discrete entities 100. After hydrolyzing the hydrogel beads, the biological samples 108, i.e. the clonal cells in this example, may be extracted from the liquid medium 106 directly.

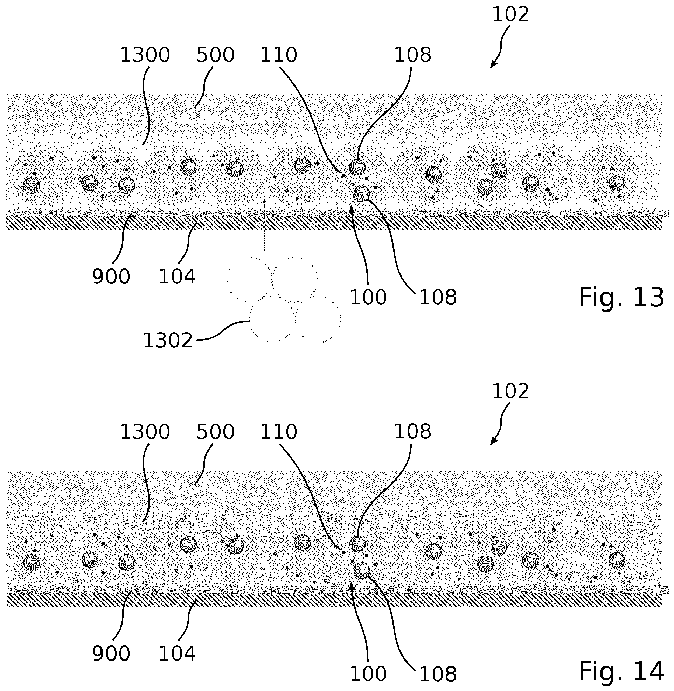

Figures 13 to 17 are schematic views of the steps of the method for assaying the plurality of biological samples 108 according to another embodiment.

The view of Figure 13 illustrates a first step of the method in which the plurality of discrete entities 100 is arranged in the liquid medium 106 inside the imaging container 102. A polymerizing agent is added to the liquid medium 106 in order to form a polymeric compound 1300 enclosing the plurality of discrete entities 100, the polymeric compound having pores 1302 with a large pore size allowing for a quick diffusion of a stimulating reagent. Alternatively, a gel may be formed from the liquid medium 106 by adding a gelling agent. The protective layer 500 may be formed above the polymeric compound and the monolayer formed by the discrete entities 100.

The view of Figure 14 illustrates a second step of the method in which the stimulating reagent is added to the polymeric compound 1300. The stimulating agent is indicated in Figure 14 by a different hatching of the polymeric compound 1300 formed from the liquid medium 106. The stimulating reagent is an experimental stimuli and causes the biological samples 108 enclosed in the discrete entities 100 to react in a certain observable way. The purpose of the method is to observe the reaction of the biological samples 108 to the stimulating reagent, and to select the biological samples 108 that react in a desired way.

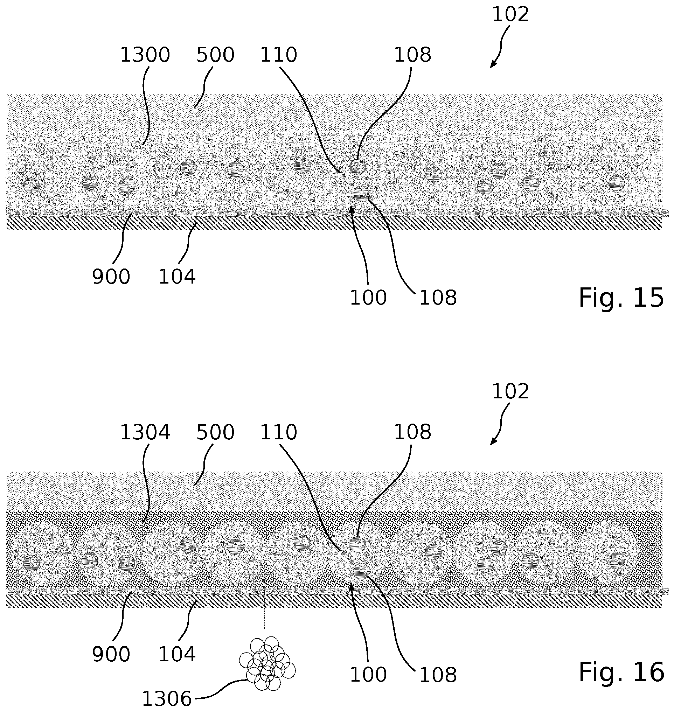

After the stimulating reagent has been added, the stimulating reagent is allowed to diffuse through the polymeric compound 1300 into the hydrogel beads of the discrete entities 100 to react with the biological samples 108. This diffusion of the stimulating reagent into the hydrogel beads of the discrete entities 100 is illustrated by the view of Figure 15, in particular by the different hatching of the discrete entities 100 which matches the hatching of the polymeric compound 1300 in Figures 14 and 15.

The view of Figure 16 illustrates a third step of the method in which another polymerizing reagent is added to the polymeric compound 1300 to form a new polymeric compound 1304 having smaller sized pores 1306 that prevent the stimulating reagent from diffusing out of the hydrogel beads. Before or after adding the polymerizing reagent, the remaining stimulating reagent may be washed out of the polymeric compound 1300, 1304.

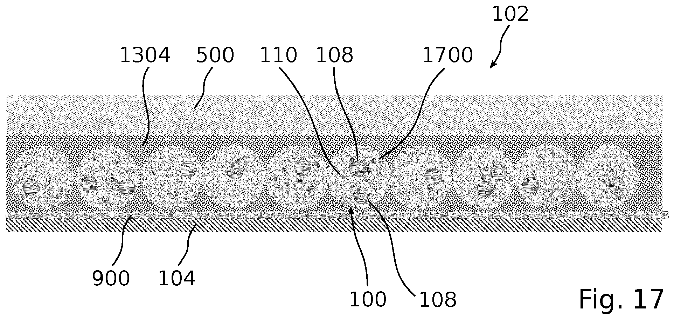

The view of Figure 17 illustrates a fourth step of the method in which an optical read-out of at least a part of the plurality of discrete entities 100 is generated. The data generated from the optical read-out is further processed to generate representation of the markers 110 of the discrete entities 100 that have been captured by the optical read-out. Further, at least one characteristic related to the biological samples 108 is determined from the data. In the present embodiment, the characteristic is the reaction of the biological samples 108 to the stimulating reagent, exemplary shown in Figure 17 as the production of antibodies 1700. From the characteristic the discrete entities of interest are then determined which may be extracted in a further step of the method.

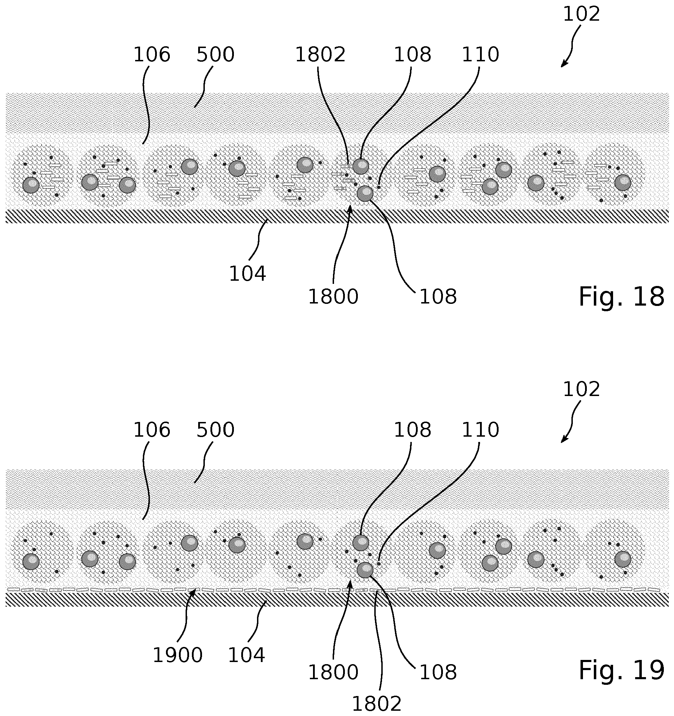

Figures 18 and 19 are schematic views of the steps of the method for assaying the plurality of biological samples 108 according to another embodiment.

In this embodiment, the biological samples 108 are enclosed in the hydrogel beads together with a compound 1802, in particular a biological compound 1802, that is sensitive to the characteristic related to the biological sample 108 in the imaging container 102, for example molecules for FirePlex, NanoArrays or OrigamiSeq assaying techniques. This is shown in the view of Figure 18. The pore size of the hydrogel beads is chosen such that the target may diffuse out of the hydrogel beads once the compound 1802 has reacted.

In the view of Figure 19, the sensitive compound 1802 has reacted and diffused out of the hydrogel beads of the discrete entities 1800. The compound 1802 has formed a floor phase 1900 at the bottom of the imaging container 102. The optical read-out captures this floor phase 1900 as well, and at least one characteristic related to the biological samples 108 is determined from the floor phase 1900 based on the optical read-out. In the view of Figure 19, a number of molecules of the compound 1802 have reacted according to the characteristic, and the discrete entities 1800 located directly above the reacted compound 1802 have been flagged as discrete entities of interest. These discrete entities 1800 may then be extracted in a further step for further processing.

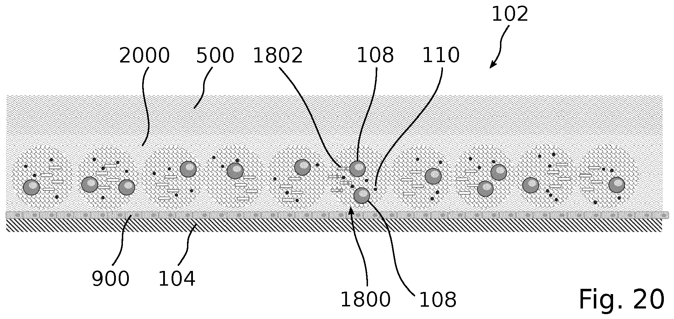

Figure 20 is a schematic views of a step of the method for assaying the plurality of biological samples 108 according to another embodiment.

In this embodiment, the discrete entities 1800 are suspended in an oil phase 2000. In other words, in this embodiment the liquid medium 106 is an oil. The oil provides the advantage that it effectively seals the discrete entities to avoid the escape of water soluble compounds and proteins from the discrete entities 100. In certain applications this may be desired and sealing using an additional leak-tight layer may not be possible, too time-consuming or expensive.

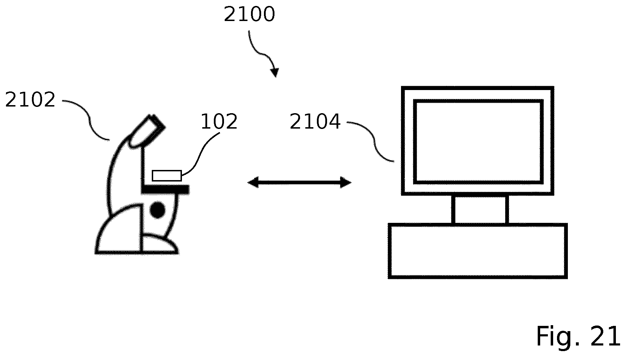

Figure 21 is a schematic view of a device 2100 for assaying a large number of biological samples 108.

The device 2100 is configured to perform the method for assaying a large number of biological samples 108 described in this document. The system comprises the imaging container 102, an optical read-out unit 2102 and a control unit 2104, exemplary formed as a computer system. The optical read-out unit 2102 is exemplary formed as a microscope and is configured to capture images of the imaging container 102 and the discrete entities 100 contained therein. The optical read-out unit 2102 is further connected to the computer system. The computer system is configured to execute at least a part of a method described herein. The computer system may be configured to execute a machine learning algorithm. The computer system and microscope may be separate entities but can also be integrated together in one common housing. The computer system may be part of a central processing system of the microscope and/or the computer system may be part of a subcomponent of the microscope, such as a sensor, an actor, a camera or an illumination unit, etc. of the microscope.

The computer system may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system may comprise any circuit or combination of circuits. In one embodiment, the computer system may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system X20 may also include a display device, one or more speakers, and a keyboard and/or controller, which can include a mouse, trackball, touch screen, voice-recognition device, or any other device that permits a system user to input information into and receive information from the computer system.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device 2100, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device 2100, a memory device 2100 or the like. The apparatus or system may, for example, comprise a file server for transferring the computer program to the receiver.

In some embodiments, a programmable logic device 2100 (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device 2100 corresponds to a method step or a feature of a method step. Analogously, aspects described in the context of a method step also represent a description of a corresponding block or item or feature of a corresponding apparatus.

### Reference signs

- 100: discrete entities
- 102: imaging container
- 104: bottom surface
- 106: liquid medium
- 108: biological sample
- 110: marker
- 400: vessel
- 500: protective layer
- 800: hydrolyzing agent
- 900: layer
- 1300: polymeric compound
- 1302: pore
- 1304: polymeric compound
- 1306: pore
- 1700: antibody
- 1800: discrete entities
- 1802: compound
- 1900: floor phase
- 2000: oil phase
- 2100: device
- 2102: optical read-out unit
- 2104: control unit

## Claims

1. Method for assaying a plurality of biological samples (108), comprising the following steps:
a) preparing a plurality of discrete entities (100), each discrete entity (100) comprising a polymeric compound, at least one of the biological samples (108) and a marker (110);
b) arranging the plurality of discrete entities (100, 1800) in an imaging container (102);
c) generating at least one first optical read-out of at least one discrete entity (100, 1800) of the plurality of discrete entities (100, 1800);
d) determining a first representation of the marker (110) and at least one characteristic related to the biological sample (108) for at least one discrete entity (100, 1800) of the plurality of discrete entities (100, 1800) based on the first optical read-out; and
e) identifying at least one discrete entity (100, 1800) of the plurality of discrete entities (100, 1800) from the imaging container (102) as a discrete entity (100, 1800) of interest based on the at least one characteristic related to the biological sample (108) and the first representation of the marker (110) of said at least one discrete entity (100, 1800).

2. Method for assaying a plurality of biological samples (108), comprising the following steps:
a) preparing a plurality of discrete entity precursors, each precursor comprising a polymeric compound and at least one of the biological samples (108) enclosed by the polymeric compound;
b) arranging the plurality of discrete entity precursors in an imaging container (102);
c) creating a plurality of discrete entities (100, 1800) from the plurality of discrete entity precursors, each discrete entity (100, 1800) comprising the polymeric compound, the biological samples (108) and a marker (110);
e) generating at least one first optical read-out of at least one discrete entity (100, 1800) of the plurality of discrete entities (100, 1800);
f) determining a first representation of the marker (110) and at least one characteristic related to the biological sample (108) for at least one discrete entity (100, 1800) of the plurality of discrete entities (100, 1800) based on the first optical read-out; and
g) identifying at least one discrete entity (100, 1800) of the plurality of discrete entities (100, 1800) from the imaging container (102) as a discrete entity (100, 1800) of interest based on the at least one characteristic related to the biological sample (108) and the first representation of the marker (110) of said at least one discrete entity (100, 1800).

3. The method according to claim 1 or 2, wherein the biological sample (108) and the marker (110) are enclosed by the polymeric compound; and/or wherein the polymeric compound comprises a marker (110) and the biological sample (108) - with or without a marker (110) marking the biological sample (108) - is enclosed by the polymeric compound.

4. The method according to any one of the previous claims, comprising the additional step of immobilizing the plurality of discrete entities (100, 1800) or the plurality of discrete entity precursors in the imaging container (102).

5. The method according to any one of the previous claims, comprising the additional step of extracting at least one discrete entity (100, 1800) of the plurality of discrete entities (100, 1800) from the imaging container (102) based on the at least one characteristic related to the biological sample (108) and the first representation of the marker (110) of said at least one discrete entity (100, 1800).

6. The method according to any one of the previous claims, comprising the additional steps of:
generating at least one second optical read-out after a predefined period of time has passed;
determining a second representation of the marker (110) and the characteristic related to the biological sample (108) for at least one discrete entity (100, 1800) of the plurality of discrete entities (100, 1800) based on the second optical read-out;
comparing the first representations and the second representations in order to identify at least one discrete entity (100, 1800) of the plurality of discrete entities (100, 1800);
comparing the characteristic related to the biological sample (108) determined from the first optical read-out to the characteristic related to the biological sample (108) determined from the second optical read-out; and
preferably extracting at least one discrete entity (100, 1800) of the plurality of discrete entities (100, 1800) from the imaging container (102) based on said comparison.

7. The method according to claim 6, comprising the additional step of cultivating the discrete entities (100, 1800), in particular the biological samples (108) being part of the discrete entities (100, 1800), between the first optical read-out and the second optical read-out.

8. The method according to any one of the previous claims, comprising the additional step of arranging a fluid or a liquid medium (106, 2000) inside the imaging container (102) before arranging the plurality of discrete entities (100, 1800) or the plurality of discrete entity precursors inside the imaging container (102).

9. The method according to claim 8, comprising the additional step of arranging a protective layer (500) atop the liquid medium (106, 2000).

10. The method according to claim 8 or 9, comprising the additional step of adding a reagent to the liquid medium (106, 2000) in order to form a gel or a polymeric compound (1300, 1304) enclosing the plurality of discrete entities (100, 1800) or the plurality of discrete entity precursors.

11. The method according to any one of the previous claims, comprising the additional step of providing a compound (1802), in particular a biological compound, that is sensitive to the characteristic related to the biological sample (108) in the imaging container (102); wherein the characteristic related to the biological sample (108) is determined based on a reaction of the compound.

12. The method according to step 11, wherein the compound is provided as a layer (900) on the bottom of the imaging container (102).

13. The method according to any one of the previous claims, wherein each marker (110) comprises at least one of a luminescent microbead, a non-fluorescent microbead, a nanoruler, and a dye.

14. The method according to any one of the previous claims, wherein each marker (110) comprises at least one element, which has a directional component, in particular a nanoruler, a rod-shaped nanostructure or an elongated microbead.

15. The method according to any one of the previous claims, wherein each marker (110) comprises a three-dimensional pattern.

16. A device (2100) for assaying a large number of biological samples (108), configured to perform the method according to any one of the previous claims, the device (2100) comprising:
an imaging container (102) configured to receive the plurality of discrete entities (100, 1800) and/or the plurality of discrete entity precursors; and
an optical read-out unit (2102) configured to perform at least one optical read-out of the plurality of discrete entities (100, 1800);
wherein a control unit (2104) is configured to at least determine a first representation of the marker (110) and at least one characteristic related to the biological sample (108) for each discrete entity (100, 1800) of the plurality of discrete entities (100, 1800) based on the at least one optical read-out performed by the optical read-out unit (2102).

17. A computer program product comprising a program code that causes the device (2100) according to claim 16 to perform the method according to any one of the claims 1 to 15 when run on a processor.

18. A computer-readable medium comprising the computer program product of claim 17.
